# EUROPEAN PATENT APPLICATION

(11) **EP 4 166 130 A1**
(43) Date of publication of application: **19.04.2023**
(21) Application number: 21820085.5
(22) Date of filing: 10.06.2021
(51) Int. Cl.: A61K 8/73, A61K 8/02, A61K 8/37, A61Q 1/14, A61Q 19/10

(54) **SCRUBBING AGENT, SCRUBBING AGENT-CONTAINING COMPOSITION, AND SCRUBBING AGENT PRODUCTION METHOD**

(30) Priority: 10.06.2020 JP 2020100652
(71) Applicant: Daicel Corporation, Osaka 530-0011 (JP)
(72) Inventor: GA, Kyokutou, Tokyo 108-8230 (JP); MATSUO, Yukiko, Tokyo 108-8230 (JP)
(74) Representative: Grünecker Patent- und Rechtsanwälte PartG mbB
(86) International application number: PCT/JP2021/022083
(87) International publication number: WO 2021/251458

(57) **Abstract**

This scrub agent is in particle form with a plurality of vertices and has a median of particle size distribution set to a value in a range of from 0.1 mm to 1.0 mm.

## Description

### Technical Field

The present disclosure relates to a scrub agent, a scrub agent-containing composition, and a method of producing a scrub agent.

### Background Art

Scrub agent-containing compositions are known as skin cleaning compositions (cleansing agents) for use to clean dirt on skin on the body, or as massaging agents for use to massage skin on the body. Examples of a scrub agent include particles obtained by processing a natural material such as walnut shell or a synthetic material such as resin, as described in Patent Document 1.

### Citation List

### Patent Document

Patent Document 1: JP 2019-189820 A

### Summary of Invention

### Technical Problem

A scrub agent is expected to, for example, stably exhibit the effect obtained by bringing the scrub agent into contact with skin on the body (hereinafter, this effect is also simply referred to as the scrubbing effect). However, depending on the configuration of the scrub agent, such effect may be difficult to obtain.

As such, an object of the present disclosure is to stably obtain a good scrubbing effect when a scrub agent is used.

### Solution to Problem

In order to solve the issue described above, a scrub agent according to one aspect of the present disclosure is in particle form with a plurality of vertices and has a median of particle size distribution set to a value in a range of from 0.1 mm to 1.0 mm.

According to the configuration described above, the scrub agent is in particle form with a plurality of vertices and has a median of particle size distribution set to a value in a range of from 0.1 mm to 1.0 mm. This allows the particle size of the scrub agent to be made uniform to a certain extent. Accordingly, an even scrubbing effect can be obtained along with a stable user experience when the scrub agent is being used. In addition, by setting the median of particle size distribution of the scrub agent to a value in the range described above, the scrub agent can be in particle form with a plurality of vertices while having a small diameter. As such, the scrub agent can be brought into wide contact with skin on the body, and skin can be easily cleaned or pressed with a small external force. As a result, a good scrubbing effect can be obtained stably.

A scrub agent-containing composition according to one aspect of the present disclosure includes any of the scrub agents described above. As a result, a scrub agent-containing composition capable of stably achieving a good scrubbing effect can be obtained.

A method of producing a scrub agent according to one aspect of the present disclosure includes: a first step of extrusion-molding a linear material by extruding a molten material containing a cellulose ester from an extrusion hole in which an edge of an opening has a maximum inner diameter set to a value in a range of from 0.4 mm to 3.0 mm; and a second step of cutting the linear material in a direction perpendicular to an extrusion direction from the extrusion hole to obtain a scrub agent in particle form with a plurality of vertices and having a median of particle size distribution set to a value in a range of from 0.1 mm to 1.0 mm.

According to the above method, the scrub agent having a desired particle shape and particle size can be produced in the second step. As such, for example, the scrub agent can be produced efficiently in a high yield without being subjected to a step of classifying the particles obtained by cutting the linear material.

### Advantageous Effects of Invention

According to each aspect of the present disclosure, when the scrub agent-containing composition is used, a good effect can be obtained evenly by the scrub agent.

### Brief Description of Drawings

FIG. 1 is a perspective view of a particle of a scrub agent according to a first embodiment.
FIG. 2 is a side view perpendicular to an axis of the particle of the scrub agent of FIG. 1.
FIG. 3 is a schematic view of an extruder according to the first embodiment.
FIG. 4 is a front view of an extrusion die of FIG. 1.
FIG. 5 is a schematic view illustrating a state in which a scrub agent-containing composition of the first embodiment is spread on the skin surface.
FIG. 6 is a perspective view of a particle of a scrub agent according to a second embodiment.
FIG. 7 is a front view of an extrusion die according to the second embodiment.
FIG. 8 is a perspective view of a particle of a scrub agent according to a first variation of the second embodiment.
FIG. 9 is a perspective view of a particle of a scrub agent according to a second variation of the second embodiment.
FIG. 10 is a perspective view of a particle of a scrub agent according to a third variation of the second embodiment.
FIG. 11 is a diagram illustrating a state in which a degree of parallelism of a particle of a scrub agent of an example is being measured.

### Description of Embodiments

Embodiments of the present invention will be described below with reference to the drawings.

### First Embodiment

### Scrub agent

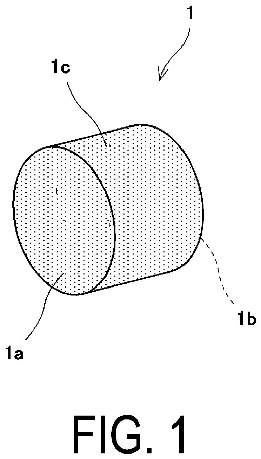

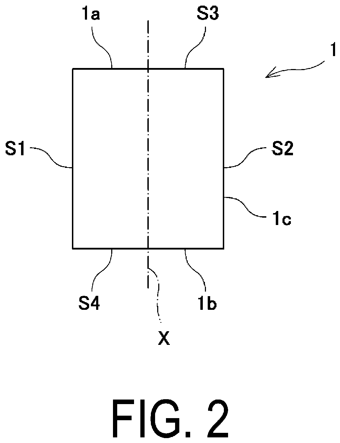

FIG. 1 is a perspective view of a particle of a scrub agent 1 according to a first embodiment. FIG. 2 is a side view perpendicular to an axis X of the particle of the scrub agent 1 of FIG. 1. The scrub agent 1 is in contact with the skin on the body of a user at the time of use. As a result, the scrub agent 1 cleans the skin by removing, for example, build-up on the skin (keratinocytes, sebum, etc.) and dirt such as dust adhering to the skin. The scrub agent 1 may be a massaging agent that exerts a blood circulation promoting effect (massaging effect) or the like when it comes into contact with the skin.

The scrub agent 1 is formed into particles with a plurality of vertices. A vertex refers to a point where three or more faces meet in a case in which the particle shape of the scrub agent 1 is a polyhedral shape. In a case in which the particle shape of the scrub agent 1 is a cylindrical shape or an elliptical cylindrical shape, a vertex refers to a boundary (edge) between an end face and the side face. In a case in which the particle shape of the scrub agent 1 is neither a polyhedron shape, a cylindrical shape, nor an elliptical cylindrical shape, a vertex refers to at least either one of a boundary (edge) between two adjacent faces or a point where three or more faces meet. As an example, the scrub agent 1 of the present embodiment has a particle shape that is a cylindrical shape or an elliptical cylindrical shape (here, a cylindrical shape). By adjusting the particle shape of the scrub agent 1, for example, the user experience of the scrub agent 1 can be adjusted.

The median of the particle size distribution (median size) of the scrub agent 1 is set to a value in a range of from 0.1 mm to 1.0 mm. This allows the particle size of the scrub agent 1 to be uniform to a certain extent. By setting the median of particle size distribution of the scrub agent 1 to a value in the range described above, the scrub agent 1 can give an improved user experience. Furthermore, changes in user experience due to the variation in the particle size of the scrub agent 1 are also suppressed. Note that the particle size mentioned here refers to the minimum value of the horizontal projected area of a particle of the scrub agent 1. Also, the particle shape of the scrub agent 1 described above includes a shape having a slight shape error that may be generated during production.

The median of particle size distribution of the scrub agent 1 can be measured, for example, based on dynamic light scattering. To give an example of this measurement method, first, the scrub agent 1 is dispersed in a liquid to prepare a dispersion. Thereafter, the dispersion is placed on a measuring device (e.g., "SK Laser Microsizer LMS-2000e" available from Seishin Enterprise Co., Ltd., ultrasonic treatment of 1 minute, refractive index of 1.52), and the volume frequency particle size distribution is measured based on laser diffraction. In this way, the median of particle size distribution of the scrub agent 1 can be measured. Note that the median of particle size distribution refers to the value (µm) of the particle size corresponding to 50% of the integrated scattering intensity in this particle size distribution.

The light transmittance of the scrub agent 1 in a wavelength region of from 380 nm to 780 nm is set to a value in a range of from 0.8% to 100%. This gives the scrub agent 1 transparency to visible light in the wavelength region described above. By having such transparency, when mixed with another component, for example, the scrub agent 1 can be unnoticeable and can have an improved degree of freedom of the design.

The scrub agent 1 also contains greater than 50 wt.% of (in other words, as the main component) a biodegradable component. The scrub agent 1 of the present embodiment contains a cellulose ester as the biodegradable component. This imparts biodegradability to the scrub agent 1, reducing the burden to the environment imposed by the scrub agent 1. Examples of the cellulose ester include cellulose acetates such as cellulose diacetate and cellulose triacetate. When a cellulose acetate is used, the equilibrium moisture ratio of the scrub agent 1 can be adjusted by adjusting the degree of acetyl group substitution. The biodegradability referred to here is, for example, marine biodegradability. The biodegradable component of the scrub agent 1 refers to a component having a marine biodegradation of 40 wt.% or greater in 90 days as measured in accordance with ASTM D6691.

The scrub agent 1 may include an additive. Examples of the additive include, for example, a plasticizer, a thermal stabilizer, a foam nucleating agent, and an auxiliary foaming agent. Because cellulose esters are not thermally meltable, a plasticizer is used in combination in a case in which a cellulose ester is used as a material of the scrub agent 1.

For plasticizers, detailed examples are found in, for example, "Handbook of Plasticizers," Ed. Wypych, George, ChemTec Publishing (2004). Examples of the plasticizer include dimethyl phthalate; diethyl phthalate; dibutyl phthalate; dioctyl phthalate; diisononyl phthalate; butyl benzyl phthalate; butyl phthalyl butyl glycolate; tris(2-ethylhexyl)trimellitate; triethyl phosphate, triphenyl phosphate, tricresyl phosphate, p-phenylene bis(diphenyl phosphate), and other phosphate derivatives; diisobutyl adipate; bis(2-ethylhexyl)adipate; triethyl citrate; acetyl triethyl citrate; plasticizers containing citric acid (e.g., Citroflex (trade name)); monoacetin; diacetin; triacetin; tripropionin; tributyrin; sucrose acetate isobutyrate; glucose pentapropionate; triethylene glycol-2-ethylhexanoate; polyethylene glycol; polypropylene glycol; polypropylene glycol dibenzoate; polyethylene glutarate; polyethylene succinate; polyalkyl glycosides; 2,2,4-trimethyl-1,3-pentanediol isobutyrate; diisobutyrate; phthalate copolymers; 1,3-butanediol; aliphatic epoxide-terminated 1,4-butanediol; bis(2-ethylhexyl)adipate; epoxidized soybean oil; and a substance selected from the group consisting of mixtures of these compounds.

Examples of the plasticizer also include a glycerin ester. Examples of the glycerin ester include a lower fatty acid ester of glycerin, in other words, an ester compound of glycerin and a fatty acid having from 2 to 4 carbons. A fatty acid having 2 carbons is acetic acid, a fatty acid having 3 carbons is propionic acid, and a fatty acid having 4 carbons is butyl acid. The glycerin ester may be an ester in which all three hydroxyl groups of glycerin are esterified with the same fatty acids, an ester in which two hydroxyl groups are esterified with the same fatty acids, or an ester in which all three hydroxyl groups of glycerin are esterified with different fatty acids.

Glycerin esters are non-toxic and easily biodegraded, and thus have a small burden to the environment. When a cellulose acetate is used as the cellulose ester serving as the main component, the glass transition temperature of a cellulose triacetate composition for thermoforming obtained by using a glycerin ester can be lowered. As such, excellent thermoformability can be imparted to a raw material, for example. When the scrub agent 1 contains a glycerin ester, the equilibrium moisture ratio of the scrub agent 1 can be adjusted by adjusting the content of the glycerin ester.

When the fatty acid is acetic acid, examples of the glycerin ester include triacetin, in which three hydroxyl groups of glycerin are esterified with acetic acid, and diacetin, in which two hydroxyl groups are esterified with acetic acid.

Among the glycerin esters described above, triacetin (glycerol trisacetate), in which three hydroxyl groups of glycerin are esterified with acetic acid (in other words, acetylated), is preferred, for example. Triacetin is a component recognized as safe even if ingested by humans and is easily biodegraded, and thus has a small burden to the environment. In addition, a cellulose acetate composition for thermoforming obtained by adding triacetin to a cellulose acetate gives the scrub agent 1 an improved biodegradability compared to a case in which cellulose acetate is used alone. Furthermore, the use of triacetin together with a cellulose acetate can efficiently lower the glass transition temperature of the cellulose acetate. As such, even better thermoformability can be imparted to a raw material, for example.

Note that for triacetin, one that is pure in terms of chemical structure and high in purity is preferred. In addition, for example, a plasticizer containing 80 wt.% or greater or 90 wt.% or greater of triacetin with the remaining being monoacetin and/or diacetin may be used.

The scrub agent 1 preferably includes, for example, the glycerin ester in a value in a range of from 10 parts by weight to 30 parts by weight per 100 parts by weight of the total amount of cellulose ester and glycerin ester. When a cellulose acetate is used as the cellulose ester, for example, the thermoformability of the scrub agent 1 can be improved by increasing the content of the glycerin ester. In addition, when a cellulose acetate is used as the cellulose ester, the prevention of bleed-out of the glycerin ester can be facilitated by reducing the content of the glycerin ester.

Further, when viewed from one direction (in the present embodiment, one direction perpendicular to the axis X of the cylindrical particle of the scrub agent 1), the scrub agent 1 has a particle shape having a contour including a pair of sides S 1 and S2 extending in a first direction perpendicular to the one direction, the pair of sides S 1 and S2 being separated in a second direction perpendicular to the first direction, a degree of parallelism of the pair of sides S 1 and S2 being set to a value in a range of from 0 mm to 0.50 mm.

Further, when viewed from the one direction, the scrub agent 1 has a particle shape having a contour including the pair of sides S 1 and S2 as the first pair of sides and further including a second pair of sides S3 and S4 extending in the second direction, the second pair of sides S3 and S4 being separated in the first direction. A degree of parallelism of the second pair of sides S3 and S4 is set to a value in a range of from 0 mm to 0.10 mm. With the two pairs of sides S 1 to S4 included, the contour of the particle of the scrub agent 1, when viewed from the one direction, is formed into a quadrangular shape. As a result, the particle of the scrub agent 1 has two pairs of surface regions having different degrees of parallelism corresponding to the two pairs of sides S 1 to S4.

In addition, the scrub agent 1 has a particle shape including a pair of faces 1a and 1b that are arranged separately from each other, a degree of parallelism of one face of the pair of faces 1a and 1b when the other face is serving as a reference plane (datum plane) being set to a value in a range of from greater than 0 mm to 0.20 mm. As a result, the one face of the particle of the scrub agent 1 is formed with a certain degree of unevenness that is allowed by the range of parallelism. The pair of faces 1a and 1b of the scrub agent 1 of the present embodiment correspond to a cut surface at which a linear material 15, which will be described below, is cut.

A degree of parallelism mentioned here is defined in JIS B 0022 and JIS B 0621:1984. That is, when geometric tolerances are instructed for a related form, a degree of parallelism indicates a magnitude of deviation of a straight line form or a planar form that is supposed to be parallel to a geometric straight line or geometric plane that is parallel to a datum straight line or a datum plane which is a theoretically correct geometric reference set to regulate the tolerance zone. The shape, the side length, and the particle size of the scrub agent 1 can be confirmed and measured using, for example, a commercially available digital microscope (e.g., "RH-2000" available from HIROX Co., Ltd.).

As described above, the scrub agent 1 of the present embodiment is in particle form with a plurality of vertices and has a median of particle size distribution set to a value in a range of from 0.1 mm to 1.0 mm. This allows the particle size of the scrub agent 1 to be made uniform to a certain extent. Accordingly, an even scrubbing effect can be obtained along with a stable user experience when the scrub agent is being used. In addition, by setting the median of particle size distribution of the scrub agent 1 to a value in the range described above, the scrub agent 1 can be in particle form with a plurality of vertices while having a small diameter. As such, the scrub agent 1 can be brought into wide contact with skin on the body, and skin can be easily cleaned or pressed with a small external force. As a result, a good scrubbing effect can be obtained stably.

The light transmittance of the scrub agent 1 in a wavelength region of from 380 nm to 780 nm is set to a value in a range of from 0.8% to 100%. This can, for example, prevent the color of a scrub agent-containing composition 20, which will be described later, from being affected by the scrub agent 1 during the production of the scrub agent-containing composition 20. As such, the scrub agent 1 can give a better scrubbing effect while the scrub agent-containing composition 20 can have an improved degree of freedom in designing the appearance.

Also, the scrub agent contains greater than 50 wt.% of a biodegradable component. This improves the biodegradability of the scrub agent 1, reducing the burden to the environment imposed by the scrub agent 1. Also, the biodegradable component includes a cellulose ester. This can give the scrub agent 1 transparency to visible light and high biodegradability.

As an example, the scrub agent 1 has a particle shape that is a cylindrical shape. Accordingly, a small vertex portion can be formed at a portion corresponding to a portion between an end face (faces 1a or 1b) and the side face of the cylindrical particle of the scrub agent 1. Thus, for example, by lightly bringing the scrub agent 1 into contact with skin or by lightly pressing the scrub agent 1 on skin, a scrubbing effect can be obtained with a small external force.

Further, when viewed from one direction, the scrub agent 1 of the present embodiment has a particle shape having a contour including the pair of sides S1 and S2 extending in a first direction perpendicular to the one direction, the pair of sides S1 and S2 being separated in a second direction perpendicular to the first direction, a degree of parallelism of the pair of sides S1 and S2 being set to a value in a range of from 0 mm to 0.50 mm.

According to this configuration, the pair of sides S1 and S2 of the scrub agent 1 are kept in parallel with high precision, and thus the particle shape of the scrub agent 1 can be easily made uniform. As a result, an even scrubbing effect can be exerted along with a stable scrubbing effect when the scrub agent 1 is being used.

Further, the scrub agent 1 of the present embodiment has a particle shape having a contour including the pair of sides S1 and S2 as the first pair of sides and further including the second pair of sides S3 and S4 extending in the second direction, the second pair of sides S3 and S4 being separated in the first direction when viewed from the one direction, a degree of parallelism of the second pair of sides S3 and S4 being set to a value in a range of from 0 mm to 0.10 mm.

As a result, for example, a scrub agent 1 having particle shape with a contour including two pairs of sides S1 to S4 having different degrees of parallelism when viewed from the one direction can be obtained. According to the scrub agent 1, for example, the characteristics of a surface corresponding to the first pair of sides S1 and S2 of the particle and a surface corresponding to the second pair of sides S3 and S4 are different from each other, and thus the scrub agent 1 can be made multifunctional. Further, a scrub agent 1 having a particle shape with a contour including two pairs of sides S1 to S4 having similar degrees of parallelism to each other can be obtained. According to the scrub agent 1, a more stable scrubbing effect can be obtained when the scrub agent 1 is being used.

In addition, the scrub agent 1 of the present embodiment has a particle shape including the pair of faces 1a and 1b that are arranged separately from each other, a degree of parallelism of one face of the pair of faces 1a and 1b when the other face is serving as a reference plane being set to a value in a range of from greater than 0 mm to 0.20 mm.

According to the configuration described above, by setting the degree of parallelism of the one face to the above value, the pair of faces 1a and 1b can be arranged in a highly parallel manner. As a result, the scrub agent 1 can exhibit a good scrubbing effect via, for example, a vertex provided on the edge of the pair of faces 1a and 1b.

In an example, the scrub agent 1 includes a glycerin ester. This makes it possible to, for example, form the scrub agent 1 into a desired shape during the production of the scrub agent 1 because the glycerin ester acts as a plasticizer.

In addition, the equilibrium moisture ratio of the scrub agent 1 of the present embodiment can be adjusted by adjusting at least either one of the degree of cellulose ester substitution or the content of glycerol ester. This makes it possible to adjust the user experience of the scrub agent 1 tailored to a skin type of the user, the affinity of the scrub agent 1 to the skin, or a moisture absorption property of the scrub agent 1. As such, the scrub agent 1 can have an improved degree of freedom of the design. Also, when the scrub agent 1 is combined with another component, the combination can be made easy by adjusting the equilibrium moisture ratio of the scrub agent 1 in accordance with a property of the another component such as hydrophilicity or hydrophobicity.

### Extruder

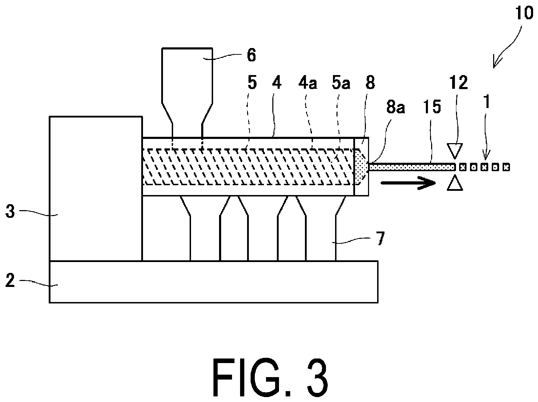

FIG. 3 is a schematic view of an extruder 10 according to the first embodiment. The scrub agent 1 is produced by, for example, the extruder 10. The extruder 10 of the present embodiment includes a drive source 2, a transmission 3, a cylindrical unit 4, at least one (here, a pair of) screw(s) 5, a hopper 6, a duct 7, an extrusion die 8, and a cutter (pelletizer) 12.

The drive source 2 generates a rotational driving force that rotates the screws 5 around its rotation axis. The transmission 3 changes the rotation speed of the output from the drive source 2 and transmits the output to the screws 5. The cylindrical unit 4 has a cylindrical internal space 4a extending in a horizontal direction. The screws 5 are pivotally supported in an internal space 4a. A helical groove 5a extending in the axial direction of the screw 5 is formed on the circumferential surface of the screw 5. The transmission 3 is disposed on one end side of the internal space 4a in the longitudinal direction, and the other end side in the longitudinal direction is open to the outside.

The hopper 6 is arranged above the cylindrical unit 4, and the lower end of the hopper 6 is connected to the internal space 4a. A raw material of the scrub agent 1 is supplied to the hopper 6. The raw material supplied to the hopper 6 is supplied to the internal space 4a. In an example, the raw material includes a cellulose ester and a plasticizer.

The duct 7 is provided to supply cooling air from a bottom of the cylindrical unit 4 to the internal space 4a. Disposed on the other end side of the internal space 4a in the longitudinal direction, the extrusion die 8 is detachably installed on the cylindrical unit 4 at the edge of the opening of the internal space 4a. The extrusion die 8 has an extrusion hole 8a communicating with the internal space 4a. The cutter 12 cuts the linear material 15 extruded from the extrusion hole 8a of the extrusion die 8 at predetermined time intervals. Note that, although the extruder 10 described here is a twin-screw extruder with a pair of screws 5, the extruder 10 may be a single-screw extruder with one screw 5, or may be another type of extruder.

When the extruder 10 is driven, a rotational driving force of the drive source 2 is transmitted to the screws 5 via the transmission 3 to rotate the screws 5. The raw material supplied to the hopper 6 is supplied to the internal space 4a and heated. As a result, the raw material becomes a molten material.

The molten material is transported by the rotating helical grooves 5a of the screws 5 toward the other end side of the internal space 4a in the longitudinal direction and is pressed to the extrusion die 8. The molten material is extruded out of the extrusion hole 8a of the extrusion die 8. At this time, the molten material is molded by the edge of the opening of the extrusion hole 8a, resulting in the linear material 15 that is solid. The linear material 15 has a cross-sectional shape corresponding to the shape of the edge of the opening of the extrusion hole 8a. The linear material 15 is cut by the cutter 12 in a direction perpendicular to the extrusion direction from the extrusion hole 8a. This results in the scrub agent 1. As such, the particles of the scrub agent 1 are molded particles formed by the extrusion hole 8a.

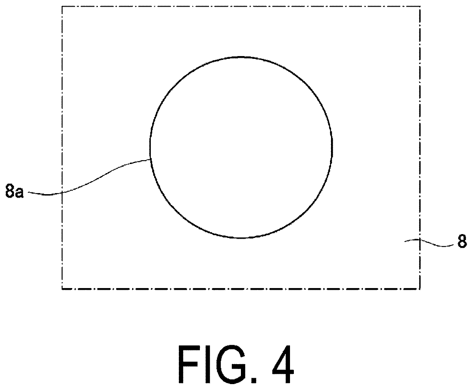

FIG. 4 is a front view of the extrusion die 8 of FIG. 3. As illustrated in FIG. 4, the extrusion hole 8a of the extrusion die 8 of the present embodiment has the edge of the opening that is formed into a circular shape. The maximum inner diameter of the extrusion hole 8a is set to a value in a range of from 0.4 mm to 0.6 mm. In the present embodiment, a scrub agent 1 that is formed into a cylindrical shape and has a median of particle size distribution set to a value in a range of from 0.1 mm to 1.0 mm can be obtained by using the extruder 10 provided with the extrusion die 8.

As such, the production method of the scrub agent 1 according to the first embodiment includes: a first step of extrusion-molding the linear material 15 by extruding a molten material containing a cellulose ester from the extrusion hole 8a in which the edge of the opening has a maximum inner diameter set to a value in a range of from 0.4 mm to 3.0 mm; and a second step of cutting the linear material 15 in a direction perpendicular to the extrusion direction from the extrusion hole 8a to obtain the scrub agent 1 in particle form with a plurality of vertices and having a median of particle size distribution set to a value in a range of from 0.1 mm to 1.0 mm.

In this way, the scrub agent 1 having a desired particle shape and particle size can be produced in the second step. Accordingly, for example, the scrub agent 1 can be produced efficiently in a high yield without being subjected to a step of classifying the particles obtained by cutting the linear material 15. Note that even when the extrusion hole 8a having an edge of the opening in which the upper limit of the maximum inner diameter of edge of the opening is set to the value above is used, the particle size of the scrub agent 1 can be adjusted to a certain degree by, for example, applying tension to the linear material 15 extruded from the extrusion hole 8a.

Also, as an example, in the first step, the molten material is extruded from the extrusion hole 8a having the edge of the opening that is circular, thereby producing the scrub agent 1 having a particle shape formed into a cylindrical shape in the second step. As a result, the scrub agent 1 that is able to stably give a good scrubbing effect and has a desired particle shape can be produced.

Furthermore, the second step of the present embodiment produces the scrub agent 1 which has, when viewed from the extrusion direction from the edge of the opening of the extrusion hole 8a, a particle shape with a contour including the pair of sides S1 and S2, a degree of parallelism of the pair of sides S1 and S2 being set to a value in a range from 0 mm to 0.50 mm. In addition, the second step produces the scrub agent 1 which has, when viewed from the extrusion direction, a particle shape with a contour including the pair of sides S3 and S4, a degree of parallelism of the pair of sides S3 and S4 being set to a value in a range from 0 mm to 0.10 mm.

Also, the second step produces the scrub agent 1 which has a particle shape including the pair of faces 1a and 1b (cut surfaces of the linear material) that are arranged separately from each other, a degree of parallelism of one face of the pair of faces 1a and 1b when the other face is serving as a reference plane being set to a value in a range of from greater than 0 mm to 0.20 mm. Furthermore, in the first step, a molten material including a glycerin ester is used.

### Preparation of raw material of scrub agent

An example of the preparation of the raw material of the scrub agent 1 will be given below. As an example, the raw material is obtained by blending a cellulose acetate (cellulose ester serving as the main component) having a degree of acetyl substitution of from 1.4 to 1.8 and a glycerin ester. Examples of the production method for the raw material include a method of directly adding the glycerin ester to the cellulose acetate.

In a case in which the glycerin ester is to be added directly to the cellulose acetate, it is preferable to mix the cellulose acetate with the glycerin ester. This mixing can be performed by a mixer such as a planetary mill, a Henschel mixer, a vibratory mill, a ball mill, or the like. A Henschel mixer is preferable because it enables homogeneous mixing and dispersion in a short period of time. In addition, although the degree of mixing is not limited, a mixing time is preferably set to, for example, from 10 minutes to 1 hour in a case in which a Henschel mixer is used.

After the cellulose acetate and the glycerin ester are mixed, the mixture is dried. Examples of the drying method include, for example, a method in which a temperature is set to 50°C or higher and 105°C or lower and the mixture is left for a period of time from 1 hour to 48 hours and dried.

The above-described mixing can be performed by a mixer such as a planetary mill, a Henschel mixer, a vibration mill, and a ball mill. In a case in which the scale of production of the scrub agent 1 is small, the mixture may be mixed using a food processor or the like. Furthermore, although mixing conditions are not limited, it is preferable to add a dispersion or a solution containing the glycerin ester to the cellulose acetate little by little while stirring the mixture. For example, a dispersion or a solution containing the glycerin ester may be added in an amount of from 2 parts by weight/min to 20 parts by weight/min per 100 parts by weight of the cellulose acetate.

A substitution degree of the cellulose acetate can be set to a value in the range from 2.2 to 2.7, for example. A substitution degree of the cellulose acetate is, for example, preferably in the range from 2.3 to 2.6, and particularly preferably a value in the range from 2.4 to 2.6. The cellulose diacetate having a degree of substitution set to such a value can be easily plasticized by using triacetin as the glycerin ester. As such, for example, by using a raw material containing cellulose diacetate and triacetin, the scrub agent 1 having a desired particle shape can be efficiently produced.

### Scrub agent-containing composition

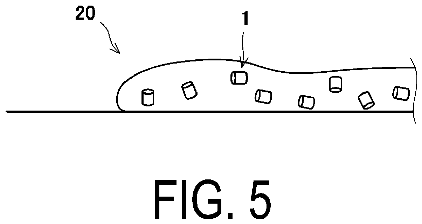

Next, the scrub agent-containing composition 20 of the present embodiment will be described. FIG. 5 is a schematic view illustrating a state in which the scrub agent-containing composition 20 of the first embodiment is spread on the skin surface. The scrub agent-containing composition 20 includes the scrub agent 1. The form of the scrub agent-containing composition 20 may be any of: a liquid preparation, such as an aqueous solution, an emulsion, and a suspension; a semi-solid preparation, such as a gel and a cream; or a solid preparation, such as a powder, a granule, and a solid. The scrub agent-containing composition 20 may be, for example, a skin cleaning composition used for cleaning skin on the body, face, or scalp. Examples of the skin cleaning composition include a facial cleaning composition, a scalp cleaning composition (shampoo), and a body cleaning composition (body wash).

When the scrub agent-containing composition 20 is a skin cleaning composition, the content of the scrub agent 1 in the scrub agent-containing composition 20 is preferably from 0.1 wt.% to 50 wt.%, more preferably from 3 wt.% to 15 wt.%, and even more preferably from 5 wt.% to 10 wt.%. By setting the content of the scrub agent 1 of the scrub agent-containing composition 20 to be in this range, for example, the particles of the scrub agent 1 can be favorably dispersed in the scrub agent-containing composition 20, and sufficient cleaning and user experience can both be achieved at the time of using the scrub agent-containing composition 20.

When the scrub agent-containing composition 20 is a skin cleaning composition, the scrub agent-containing composition 20 may include a component used in a known skin cleaning composition. Examples of the component include: a surfactant (such as an anionic surfactant, a cationic surfactant, an amphoteric surfactant, and a non-ionic surfactant), a polyhydric alcohol such as glycerin, fats and oils such as vegetable oil, a hydrocarbon such as squalane, a higher fatty acid such as lauric acid and myristic acid, a higher alcohol such as lauryl alcohol, a silicone such as dimethylpolysiloxane which is a chain polysiloxane, a preservative, a thickener, a sequestrant, a polymer such as a water-soluble polymer, a UV absorber, a UV blocker, a moisturizer such as hyaluronic acid and an amino acid, a fragrance, a pH regulator, a pearling agent, a desiccant, a vitamin, a skin activator, a blood circulation promoter, a normal bacteria controlling agent, an active oxygen removing agent, an antiinflammatory agent, a whitening agent, a bactericide, and a bioactive ingredient. Another embodiment will be described below focusing on differences from the first embodiment.

### Second Embodiment

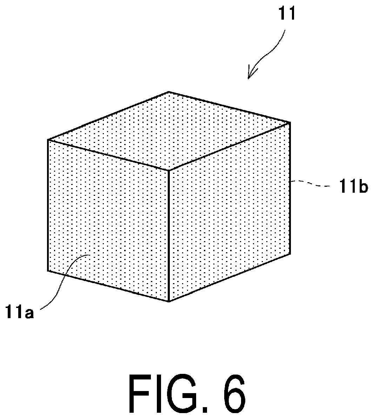

FIG. 6 is a perspective view of a particle of a scrub agent 11 according to a second embodiment. As shown in FIG. 6, the scrub agent 11 has a particle shape that is a polyhedral shape having a plurality of faces, each being a polygon. The plurality of faces include faces having any shape of a triangular, square, pentagonal, and hexagonal shape in plan view. As an example, the scrub agent 11 of the present embodiment has a hexahedral particle shape. The plurality of faces include a quadrangle face in plan view. The particle of the scrub agent 11 has a pair of faces 11a and 11b (that is, a cut surface at which the linear material 15 is cut) corresponding to the pair of faces 1a and 1b of the particle of the scrub agent 1. The pair of faces 11a and 11b are quadrangular.

As such, according to the present embodiment, the scrub agent 11 is formed into a particle shape that is a polyhedral shape having a plurality of faces, each being a polygon. In another example, the plurality of faces include faces having any one of a triangular, square,, and a hexagon in plan view. As such, many vertices can be provided in the edge portions of each face of the particle of the scrub agent 11. Accordingly, a scrub agent 11 capable of exhibiting an effective scrubbing effect can be obtained.

In addition, the scrub agent 11 of the present embodiment has a hexahedral particle shape. As such, for example, it is possible to produce the scrub agent 11 by cutting the linear material 15 having a quadrangular cross-section. Thus, the scrub agent 11 having an excellent scrubbing effect can be efficiently produced.

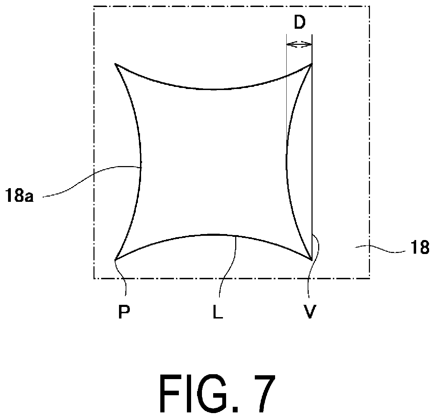

FIG. 7 is a front view of an extrusion die 18 according to the second embodiment. As illustrated in FIG. 7, an extrusion hole 18a of the extrusion die 18 of the second embodiment has an edge of the opening formed into a non-circular shape. In an example, the edge of the opening of the extrusion hole 18a is formed into a polygonal shape that has a plurality of points P, each pair of the adjacent points P being connected with a curved line segment L bending toward the center of the opening.

In a front view of the extrusion hole 8a, a maximum distance D between a curved line segment L and an imaginary line V passing through adjacent points P in the direction perpendicular to the imaginary line V is set to a value in a range of greater than 0% to 25% of the maximum inner diameter of the extrusion die 18. The edge of the opening of the extrusion hole 18a is formed into a shape having from 3 to 6 points P. Here, as an example, the edge of the opening of the extrusion hole 18a is formed into a shape having 4 points P.

Here, when the molten material is extruded from the extrusion hole 18a of the extrusion die 18, a die swell phenomenon (stress relaxation phenomenon), in which the molten material that has been compressed at the edge of the extrusion hole 18a is released and expands in the radial direction of the extrusion hole 18a, may occur. In a case in which the die swell phenomenon occurs, when the cross-sectional shape of the linear material 15 is, for example, a polygonal shape, it becomes difficult to set the cross-sectional shape to a desired shape.

To solve this issue, in the present embodiment, the edge of the opening of the extrusion hole 18a is formed into a polygonal shape having a plurality of points P in which each pair of the adjacent points P are connected with the curved line segment L; as such, the influence of the die swell phenomenon can be suppressed, and the scrub agent 11 having a desired shape can be formed easily.

In a first step according to the production method of the scrub agent 11, the molten material is extruded from the extrusion hole 18a in which the edge of the opening has a shape having a plurality of points P, thereby producing the scrub agent 11 formed into a particle shape that is a polyhedral shape having a plurality of faces, each being a polygon in a second step. As a result, the scrub agent 11 that is able to stably give a good scrubbing effect and has a desired particle shape can be produced.

In another example, in a first step, the molten material is extruded from the extrusion hole 18a in which the edge of the opening has a shape formed by connecting each pair of the adjacent points P with the curved line segment L bending toward the center of the opening. Accordingly, the influence of the die swell phenomenon that can occur when the molten material is extruded from the extrusion hole can be suppressed, and the scrub agent 11 can be formed properly.

In yet another example, in a first step, the molten material is extruded from the extrusion hole 18a in which the edge of the opening has a maximum distance D set to a value in a range of from a value greater than 0% to 25% of the maximum inner diameter of the extrusion die 18. This makes it possible to suppress the influence of the die well phenomenon, and the particles of the scrub agent 11 can be formed more easily. Hereinafter, variations of the second embodiment will be described.

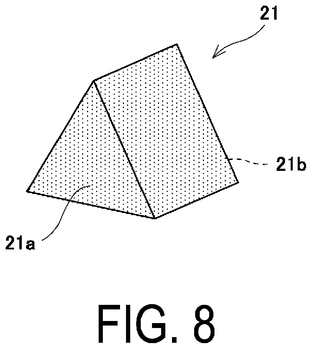

FIG. 8 is a perspective view of a particle of a scrub agent 21 according to a first variation of the second embodiment. The scrub agent 21 is formed into a triangular prism shape. The scrub agent 21 has a pair of faces 21a and 21b corresponding to the pair of faces 1a and 1b of the scrub agent 1. The pair of faces 21a and 21b have a triangular shape. The scrub agent 21 is produced by cutting the linear material 15 extruded from an extrusion hole in which the edge of the opening has three points P.

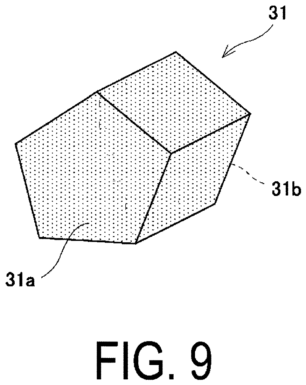

FIG. 9 is a perspective view of a particle of a scrub agent 31 according to a second variation of the second embodiment. The scrub agent 31 is formed into a pentagonal prism shape. The scrub agent 31 has a pair of faces 31a and 31b corresponding to the pair of faces 1a and 1b of the scrub agent 1. The pair of faces 31a and 3 1b have a pentagonal shape. The scrub agent 31 is produced by cutting the linear material 15 extruded from an extrusion hole in which the edge of the opening has five points P.

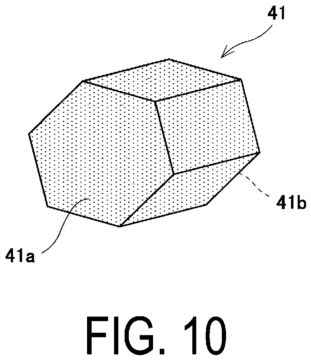

FIG. 10 is a perspective view of a particle of a scrub agent 41 according to a third variation of the second embodiment. The scrub agent 41 is formed into a hexagonal prism shape. The scrub agent 41 has a pair of faces 41a and 41b corresponding to the pair of faces 1a and 1b of the scrub agent 1. The pair of faces 41a and 41b have a hexagonal shape. The scrub agent 41 is produced by cutting the linear material 15 extruded from an extrusion hole in which the edge of the opening has six points P. The scrub agents 21, 31, and 41 also have the same effect as the scrub agent 11.

### Confirmation Test

A confirmation test will be described next, but the present disclosure is not limited to each Example described below. The scrub agents 1 and 11 of Examples 1 and 2 were produced using the following method.

### Preparation of raw material

As a cellulose ester, a cellulose acetate having an acetyl substitution degree of 2.45 (cellulose acetate produced by Daicel Corporation having a limiting viscosity of 84 mPa s) was used. The cellulose acetate was dried in a granular form for one hour in a dryer set to a temperature of 105°C. Then, the cellulose acetate was allowed to cool in a desiccator at room temperature (25°C) for 1 hour.

Then, 80 parts by weight (400 g) of the cellulose acetate was added to a mixer and stirred. During the stirring, 20 parts by weight (100 g) of triacetin was added as a plasticizer to the mixer at a rate of about 15 g/min using a pipette to be mixed with the cellulose acetate. At the time of the mixing, the mixer was stopped when half of the total amount of the triacetin was put into the mixer, and the mixture attached to the inner wall, the bottom wall, and the stirring blades of the mixer was removed. Thereafter, the remaining amount of the triacetin was added to the mixer and mixed at the above-described rate. Thereafter the mixer was stopped to remove the mixture attached to the inner wall, the bottom wall, and the stirring blades of the mixer, and the mixer was operated again for one minute or longer. As a result, a mixture was obtained. The mixture was placed in a vat and dried at 80°C for two hours. Then, the size of mass in the mixture was adjusted, and the raw material was prepared.

### Production of scrub agent

The first and second steps were performed using the extruder 10 ("Process 11" available from Thermo Fisher Scientific Inc. with the extrusion die 8 installed), resulting in, as Example 1, the scrub agent 1 including 80 wt.% of cellulose acetate and 20 wt.% of triacetin and having a cylindrical particle shape in which the design dimensions were a length of 0.5 mm in the direction of the axis X and a diameter of 0.5 mm of an end face. The extruder 10 was set as follows to produce the scrub agent 1.

Feed amount of raw material into internal space 4a: approximately 7 g/min
Heating temperature of raw material: a value in the range from 180°C to 220°C
Rotation speed of screw 5: 90 rpm
Formation speed of linear material 15: 2.5 m/min

Further, the first and second steps were performed in the same manner as in the first embodiment except that the extrusion die 18, in which the shortest distance between two adjacent points P was set to 0.5 mm, was installed on the extruder 10, resulting in, as Example 2, the scrub agent 11 including 80 wt.% of cellulose acetate and 20 wt.% of triacetin and having a hexahedral particle shape in which the design dimension was a side length of 0.5 mm.

In addition, a scrub agent that is commercially available pulverized particles of cellulose acetate having a median of particle size distribution of 0.5 mm ("CelluloScrub 500" available from ESSENTIAL CRICTERIA) was prepared as a comparative example.

### Test 1: Confirmation of particle size distribution

Next, each of the scrub agents of Example 1, Example 2, and the comparative example was classified using test sieves (JIS standard-compliant plain woven wire mesh sieves with opening sizes set to 0.318 mm, 0.385 mm, 0.5 mm, 0.6 mm, and 0.71 mm) in compliance with JIS Z 8801. As a result, particles having a particle size of less than 0.318 mm, 0.318 mm or greater and less than 0.385 mm, 0.385 mm or greater and less than 0.5 mm, 0.5 mm or greater and less than 0.6 mm, 0.6 mm or greater and less than 0.71 mm, and 0.71 mm or greater were classified. Then, the particle size distribution of each scrub agent was confirmed by measuring the weight of the classified particles of each of the scrub agents of Example 1, Example 2, and the comparative example. The results are presented in Table 1.

**[Table 1]**

| Particle Size Distribution | Example 1 (Cylindrical scrub agent) | | Example 2 (Hexahedral scrub agent) | | Comparative Example (Amorphous scrub agent) | |
|---|---|---|---|---|---|---|
| | Weight (g) | Percentage (wt.%) | Weight (g) | Percentage (wt.%) | Weight (g) | Percentage (wt.%) |
| Less than 0.318 mm | 0.0031 | 0.03 | 0.0042 | 0.041 | 0.8655 | 8.68 |
| 0. 318 mm or greater and less than 0.385 mm | 0.1129 | 1.12 | 0.1313 | 1.275 | 3.028 | 30.37 |
| 0.385 mm or greater and less than 0.5 mm | 1.3134 | 13.07 | 2.3519 | 22.838 | 5.4418 | 54.58 |
| 0.5 mm or greater and less than 0.6 mm | 8.3321 | 82.94 | 7.4333 | 72.182 | 0.5825 | 5.84 |
| 0.6 mm or greater and less than 0.71 mm | 0.2234 | 2.22 | 0.2651 | 2.574 | 0.0519 | 0.52 |
| 0.71 mm or greater | 0.0607 | 0.60 | 0.1122 | 1.090 | 0.0004 | 0.00 |
| | 10.456 (Total) | | 10.298 (Total) | | 9.9701 (Total) | |

As presented in Table 1, the particles having a value of particle size distribution in the range of from 0.385 mm to 0.6 mm were 96.01 wt.% in Example 1 and 95.02 wt.% in Example 2. This confirmed that both Examples 1 and 2 had a relatively uniform particle size. In particular, it was confirmed that Examples 1 and 2 contained a large amount of particles having a particle size of 0.5 mm. Furthermore, the results of the present test confirmed that the scrub agents 1 and 11 of Examples 1 and 2 had a median of particle size distribution in the range of from 0.1 mm to 1.0 mm (more specifically, from 0.4 mm to 0.6 mm). In comparison, the scrub agent of the comparative example was pulverized particles formed by pulverizing solid cellulose acetate. As such, it was confirmed that the scrub agent of the comparative example had a much wider particle size distribution than that of Examples 1 and 2, contained a considerable amount of particles of various particle sizes, and had a median different from that of Examples 1 and 2.

### Test 2: Confirmation of degree of parallelism of particle

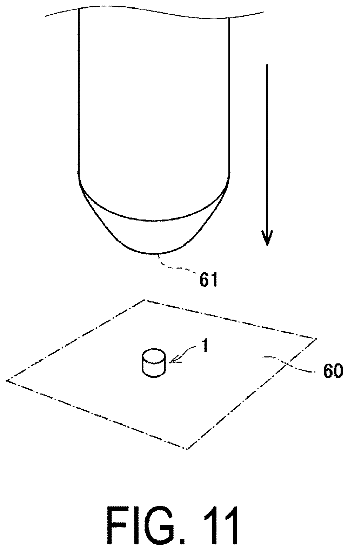

Using a digital microscope ("RH-2000" available from Hirox Co., Ltd.), a 3D image of a particle of each of the scrub agents of Example 1, Example 2, and the comparative example was obtained. FIG. 11 is a diagram illustrating a state in which a degree of parallelism of a particle of the scrub agent 1 of Example 1 is being measured.

As illustrated in FIG. 11, a particle of the scrub agent 1 or the scrub agent 11 was placed on a top surface of a stage 60 disposed horizontally, and an objective lens 61 of the microscope was brought close to the particle of the scrub agent 1 or the scrub agent 11 from one direction (the vertical direction and the extrusion direction from the extrusion hole 8a), and the particle of the scrub agent 1 or the scrub agent 11 viewed from the one direction was imaged at a predetermined focal length. A 3D image of the particle of the scrub agent 1 and 11 was obtained in this way. Based on the 3D image, the cross-sectional shape perpendicular to a horizontal plane of the scrub agent 1 or 11 was calculated.

The results confirm that the scrub agent 1 of Example 1 had a pair of faces 1a and 1b that are arranged separately from each other while the scrub agent 11 of Example 2 had a pair of faces 11a and 11b that are arranged separately from each other, a degree of parallelism of one face (the upper surface in FIG. 11) of each of the pairs of faces when the other face is serving as a reference plane being set to a value in a range of from greater than 0 mm to 0.10 mm. In contrast, the particle of the scrub agent of the comparative example did not have the above-described pair of faces, making it difficult to measure the degree of parallelism of the particle. The pair of faces 1a and 1b of the particle of the scrub agent 1 of Example 1 and the pair of faces 11a and 11b of the particle of the scrub agent 11 of Example 2 had a higher degree of parallelism, indicating that the particles of the scrub agents 1 and 11 of Examples 1 and 2 had a uniform shape.

### Test 3: Sensory evaluation of friction

Next, seven testers of a wide range of ages from 20s to 50s performed a sensory evaluation on the evenness of the sensation (grittiness, smoothness, and resistance) received from the scrub agent on a four-level scale. Specifically, the testers placed the scrub agents of Examples 1, Example 2, and the comparative example (approximately 0.2 g each) in their hands and gave evaluation based on the sensation they felt when they rubbed both hands together as if they were removing dirt from their palms. In this evaluation, A4 means a tester feels the evenness is at the highest level, A3 means the level below A4, A2 means the level below A3, and A1 means the level below A2. A4 was scored as 4 points, A3 as 3 points, A2 as 2 points, and A1 as 1 point. In this way, each of Example 1, Example 2, and the comparative examples was evaluated. The evaluation results are presented in Table 2.

**Table 2**

| | Evaluation of Evenness of Sensation Felt from Scrub Agent | | | | | | | Total Evaluation Score |
|---|---|---|---|---|---|---|---|---|
| | Evaluator 1 | Evaluator 2 | Evaluator 3 | Evaluator 4 | Evaluator 5 | Evaluator 6 | Evaluator 7 | |
| Example 1 | A4 | A3 | A4 | A3 | A3 | A2 | A4 | 23 |
| Example 2 | A2 | A3 | A3 | A3 | A3 | A3 | A3 | 20 |
| Comparative Examples | A1 | A3 | A1 | A3 | A2 | A3 | A2 | 15 |

As presented in Table 2, the scrub agents of Examples 1 and 2 were evaluated as giving a user an even feeling when being used as compared to the scrub agent of the comparative example. The reason for this evaluation is considered to be that each of Examples 1 and 2 have a relatively uniform particle size and particle shape, as described above. Further, Example 1 was evaluated to give a user a more even feeling when being used as compared to Example 2.

### Test 4: Light transmission evaluation

Next, the light transmittance of the scrub agents of Example 1, Example 2, and the comparative example was measured using a ultra violet-visible spectrophotometer (UV/VIS spectrophotometer) ("UVmini-1230" available from SHIMADZU Co., Ltd.). The measurement conditions were set as follows.
Measurement mode: T% [(transmittance measurement mode (light transmission)]
Scan range: From 380 nm to 780 nm (visible light wavelength region)
Display range: From 0 to 100%
Integrated time: 0.1 sec
Scan speed: Medium speed (equivalent to 200 nm/min in the measurement range of visible light wavelength)

The measurement results are presented in Table 3.

**[Table 3]**

| Wavelength (nm) | Transmittance (%) | | |
|---|---|---|---|
| | Example 1 | Example 2 | Comparative Examples |
| 780 | 1.8 | 1.4 | 0 |
| 570 | 1.8 | 1.4 | 0 |
| 560 | 1.8 | 1.4 | 0 |
| 480 | 1.7 | 1.2 | 0 |
| 470 | 1.7 | 1.2 | 0 |
| 440 | 1.6 | 1.1 | 0 |
| 410 | 1.5 | 1 | 0 |
| 390 | 1.3 | 0.9 | 0 |
| 380 | 1.3 | 0.8 | 0 |

As presented in Table 3, it was confirmed that Examples 1 and 2 had a transparency of transmittance of at least 0.8% or greater with respect to light having a wavelength within the measured scan range (from 380 nm to 780 nm). Each of the measured values of Examples 1 and 2 within the present test range had an average value of 1.2% or greater. In contrast, the comparative example had a transmittance of zero with respect to light having the wavelength, confirming that the comparative example was opaque.

### Test 5: Evaluation of coefficient of friction

Next, scrub agent-containing compositions each including each of the scrub agents of Example 1, Example 2, and the comparative example were prepared, and the coefficient of dynamic friction between each scrub agent-containing composition and an imitation skin was measured. The measurement conditions were set as follows. First, each of the scrub agents of Example 1, Example 2, and the comparative example was mixed with a commercially available synthetic household dish detergent ("Mama Lemon" available from Lion Corporation) and water at a weight ratio of 1:1:8, resulting in a scrub agent-containing composition A containing the scrub agent of Example 1, a scrub agent-containing composition B containing the scrub agent of Example 2, and a scrub agent-containing composition C containing the scrub agent of the comparative example.

Then, a piece of commercially available imitation skin ("BIOSKIN" available from Beaulax Co., Ltd.) was placed on a sample table of a static and dynamic friction measuring device ("Handy Tribomaster Type TL201Ts" available from Trinity-Lab Inc.). The scrub agent-containing compositions A to C were individually placed between the imitation skin and a contact probe of the measuring device, and the coefficient of dynamic friction was measured for each of the scrub agent-containing compositions. The settings of the static and dynamic friction measuring device at this time were as follows.
Load of contact probe (vertical load): 30 g or 100 g
Moving speed: 30 mm/sec
Moving distance: 60 mm
Contact probe reciprocating mode: ON
Operating time: 4 sec

Note that in this evaluation, the load of the contact probe was set to 30 g assuming a scenario in which a user uses the scrub agent-containing composition to wash their hands. Further, the load of the contact probe was set to 100 g assuming a scenario in which a user uses the scrub agent-containing composition to wash their feet.

As a result of testing at the above settings, it was confirmed that among the scrub agent-containing compositions A to C, regardless of whether the load of the contact probe was 30 g or 100 g, the fluctuation range of the coefficient of dynamic friction of the composition A containing the scrub agent 1 of Example 1 was the smallest, and the fluctuation range of the coefficient of dynamic friction of the composition B containing the scrub agent 11 of Example 2 was the second smallest after the scrub agent-containing composition A. Also, it was confirmed that the scrub agent-containing composition C of the comparative example, a composition containing the scrub agent that is amorphous pulverized particles, had a fluctuation range of the coefficient of dynamic friction that is smaller than that of the scrub agent-containing composition A and equal to or larger than that of the scrub agent-containing composition B.

This result confirms that, according to Examples 1 and 2, even when the particle sizes of the scrub agents are the similar, the fluctuation range of the coefficient of dynamic friction can be adjusted by changing the particle shape. As a result, it is conceivable that, for example, by changing the particle shape while maintaining the median of particle size distribution of the scrub agent, the degree to which the scrubbing effect is exhibited can be adjusted, and the degree of freedom in designing the scrub agent can be improved. Note that since the scrub agent of the comparative example is amorphous pulverized particles, it is difficult to change the particle shape while maintaining the median of particle size distribution, and thus it is difficult to achieve such an effect.

### Test 6: Evaluation of equilibrium moisture ratio

Next, samples No. 1 to 14 (Examples) that are scrub agents having a cylindrical shape were produced using the same production method as in Example 1 except for using triacetin as the plasticizer and using a raw material in which the amount of the plasticizer of a scrub agent immediately after production was set to a design value that is one of the values shown in Table 4. Also, samples No. 15 to 23 (Examples) that are scrub agents having a cylindrical shape were produced in the same production method as in Example 1 except for using diacetin as the plasticizer and using a raw material in which the amount of the plasticizer of a scrub agent immediately after production was set to a design value that is one of the values shown in Table 5. For comparison, sample No. 24 that is a scrub agent containing no plasticizer (similar to that of Comparative Example 1) was prepared.

The scrub agents of the samples No. 1 to 24 were conditioned by being placed in a room, adjusted to a temperature of 23°C and a humidity of 60 RH% by a precision air conditioner, for six hours or more. Thereafter, based on the dry weight method, the equilibrium moisture ratio of each of the scrub agents of samples No. 1 to 24 was measured for three times using a halogen moisture analyzer ("HB43-S" available from Mettler Toledo, LLC). The results are presented in Tables 4 to 6. "n1 to n3" in the tables indicate individually measured values, while "avg." indicates the average value of the measured values of n1 to n3.

**[Table 4]**

| Sample of Cylindrical Scrub Agent Plasticized with Plasticizer (Triacetin) | | | | | | | | | | | | | | | |
|---|---|---|---|---|---|---|---|---|---|---|---|---|---|---|---|
| Sample No. | | 1 | 2 | 3 | 4 | 5 | 6 | 7 | 8 | 9 | 10 | 11 | 12 | 13 | 14 |
| Equilibrium Moisture Ratio (wt.%) | n1 | 0.93 | 0.72 | 0.91 | 0.94 | 1.20 | 1.22 | 1.68 | 1.57 | 1.23 | 1.74 | 1.20 | 1.64 | 1.78 | 2.01 |
| | n2 | 1.02 | 0.78 | 0.98 | 0.89 | 1.09 | 1.29 | 1.36 | 1.61 | 1.30 | 1.57 | 1.35 | 1.68 | 2.01 | 2.16 |
| | n3 | 0.87 | 0.86 | 0.90 | 1.02 | 1.22 | 1.14 | 1.22 | 1.39 | 0.98 | 1.45 | 1.59 | 1.61 | 1.89 | 2.12 |
| | avg. | 0.94 | 0.79 | 0.93 | 0.95 | 1.17 | 1.22 | 1.42 | 1.52 | 1.17 | 1.59 | 1.38 | 1.64 | 1.89 | 2.11 |
| Plasticizer (Triacetin) Content (wt.%) (Design Value) | | 15 | 16 | 16 | 16 | 16 | 16 | 18 | 20 | 21 | 22 | 23 | 27 | 30 | 32 |

**[Table 5]**

| Sample of Cylindrical Scrub Agent Plasticized with Plasticizer (Diacetin) | | | | | | | | | | |
|---|---|---|---|---|---|---|---|---|---|---|
| Sample No. | | 15 | 16 | 17 | 18 | 19 | 20 | 21 | 22 | 23 |
| Equilibrium Moisture Ratio (wt.%) | n1 | 2.22 | 2.93 | 3.24 | 3.02 | 3.31 | 3.46 | 3.73 | 3.55 | 3.77 |
| | n2 | 2.66 | 3.08 | 2.99 | 3.37 | 2.83 | 3.64 | 3.55 | 3.14 | 3.76 |
| | n3 | 2.83 | 3.26 | 2.87 | 3.58 | 3.38 | 3.01 | 3.33 | 3.24 | 3.55 |
| | avg. | 2.57 | 3.09 | 3.03 | 3.32 | 3.17 | 3.37 | 3.54 | 3.31 | 3.69 |
| Plasticizer (Diacetin) Content (wt.%) (Design Value) | | 10 | 12 | 13 | 14 | 15 | 16 | 16 | 18 | 18 |

**[Table 6]**

| Sample of Scrub Agent of Comparative Example (No Plasticizer) | | |
|---|---|---|
| Sample No. | | 24 |
| Equilibrium Moisture Ratio (wt.%) | n1 | 5.81 |
| | n2 | 5.49 |
| | n3 | 6.01 |
| | avg. | 5.77 |
| Plasticizer Content (wt.%) | | 0 |

As presented in Tables 4, 5, it was confirmed that, within the test range, the equilibrium moisture ratio of the scrub agents of the samples No. 1 to 23 that are of the Examples increased when the amount of plasticizer increased, and the equilibrium moisture ratio of the scrub agents of the samples No. 1 to 23 that are of the Examples decreased when the amount of plasticizer decreased. In addition, it was confirmed that when diacetin was used as the plasticizer, the equilibrium moisture ratio increased more than when triacetin was used as the plasticizer.

From the test results, it is conceivable that, when a glycerin ester such as triacetin or diacetin is used as a plasticizer in the raw material in the Examples, the equilibrium moisture ratio of the scrub agents can be adjusted at least within a certain range by adjusting the type and amount of the glycerin ester to be added. In the Examples, in a case in which a glycerin ester such as triacetin and diacetin is used as the plasticizer and the plasticizer amount is set to a range of from 10 wt.% to 30 wt.% as a design value of a scrub agent immediately after production, it is conceivable that the equilibrium moisture ratio of the scrub agent can be set to a value within a range of from 1 wt.% to 5 wt.%. Note that, as presented in Table 6, in a scrub agent that does not contain a plasticizer, it goes without saying that the equilibrium moisture ratio of the scrub agent cannot be adjusted by changing the plasticizer amount. From the above test results, the superiority of each Example was confirmed.

Note that each of the configurations, combinations thereof, or the like in each of the embodiments are examples, and additions, omissions, replacements, and other changes to the configurations may be made as appropriate without departing from the spirit of the present disclosure. The present disclosure is not limited by the embodiments and is limited only by the claims. Each aspect disclosed in the present specification can be combined with any other feature disclosed herein.

### Reference Signs List

1, 11, 21, 31, 41 Scrub agent
8a, 18a Extrusion hole
15 Linear material
20 Scrub agent-containing composition

## Claims

1. A scrub agent in particle form with a plurality of vertices, the scrub agent having a median of particle size distribution set to a value in a range of from 0.1 mm to 1.0 mm.

2. The scrub agent according to claim 1, wherein a light transmittance in a wavelength region of from 380 nm to 780 nm is set to a value in a range of from 0.8% to 100%.

3. The scrub agent according to claim 1 or 2, comprising greater than 50 wt.% of a biodegradable component.

4. The scrub agent according to claim 3, wherein the biodegradable component includes a cellulose ester.

5. The scrub agent according to any one of claims 1 to 4, wherein a particle shape is a cylindrical shape.

6. The scrub agent according to any one of claims 1 to 4, wherein the particle shape is a polyhedral shape having a plurality of faces, each being a polygon.

7. The scrub agent according to claim 6, wherein the plurality of faces include faces having any one of a triangular, square, pentagonal, and hexagonal shape in plan view.

8. The scrub agent according to any one of claims 1 to 7, wherein the scrub agent has a particle shape having a contour including a pair of sides extending in a first direction perpendicular to the one direction, the pair of sides being separated in a second direction perpendicular to the first direction when viewed from one direction, a degree of parallelism of the pair of sides being set to a value in a range of from 0 mm to 0.50 mm.

9. The scrub agent according to claim 8, wherein the scrub agent has a particle shape having a contour including the pair of sides as the first pair of sides and further including a second pair of sides extending in the second direction, the second pair of sides being separated in the first direction when viewed from the one direction, a degree of parallelism of the second pair of sides being set to a value in a range of from 0 mm to 0.10 mm.

10. The scrub agent according to claim 8 or 9, wherein the scrub agent has a particle shape including the pair of faces that are arranged separately from each other, a degree of parallelism of one face of the pair of faces when the other face is serving as a reference plane being set to a value in a range of from greater than 0 mm to 0.20 mm.

11. The scrub agent according to any one of claims 1 to 10, wherein the scrub agent comprises a glycerin ester.

12. A scrub agent-containing composition comprising the scrub agent according to any one of claims 1 to 11.

13. A method of producing a scrub agent, the method comprising:
a first step of extrusion-molding a linear material by extruding a molten material containing a cellulose ester from an extrusion hole in which an edge of an opening has a maximum inner diameter set to a value in a range of from 0.4 mm to 3.0 mm; and
a second step of cutting the linear material in a direction perpendicular to an extrusion direction from the extrusion hole to obtain a scrub agent in particle form with a plurality of vertices and having a median of particle size distribution set to a value in a range of from 0.1 mm to 1.0 mm.

14. The method of producing a scrub agent according to claim 13, wherein in the first step, a molten material is extruded from the extrusion hole having the edge of the opening that is circular, thereby producing the scrub agent having a particle shape formed into a cylindrical shape in the second step.

15. The method of producing a scrub agent according to claim 13, wherein in the first step, a molten material is extruded from the extrusion hole in which the edge of the opening has a shape having a plurality of points, thereby producing the scrub agent having a particle shape formed into a polyhedral shape having a plurality of faces, each being a polygon, in the second step.

16. The method of producing a scrub agent according to claim 15, wherein in the first step, a molten material is extruded from the extrusion hole in which the edge of the opening has a shape formed by connecting each pair of the adjacent points with a curved line segment bending toward the center of the opening.

17. The method of producing a scrub agent according to claim 16, wherein in the first step, a molten material is extruded from the extrusion hole in which the edge of the opening has, in front view of the extrusion hole, a maximum distance between a curved line segment and an imaginary line passing through adjacent points in a direction perpendicular to the imaginary line set to a value in a range of greater than 0% to 25% of the maximum inner diameter.

18. The method of producing a scrub agent according to any one of claims 13 to 17, wherein the second step produces the scrub agent in particle form having a contour including, when viewed from an extrusion direction from the edge of the opening, a pair of sides extending in a first direction perpendicular to the extrusion direction, the pair of sides being separated in a second direction perpendicular to the first direction, a degree of parallelism of the pair of sides being set to a value in a range of from 0 mm to 0.50 mm.

19. The method of producing a scrub agent according to claim 18, wherein the second step produces the scrub agent in particle form having a contour including the pair of sides as a first pair of sides and further including a second pair of sides extending in the second direction, the second pair of sides being separated in the first direction when viewed from the extrusion direction, a degree of parallelism of the second pair of sides being set to a value in a range of from 0 mm to 0.10 mm.

20. The method of producing a scrub agent according to claim 18 or 19, wherein the second step produces the scrub agent in particle form including the pair of faces that are arranged separately from each other, a degree of parallelism of one face of the pair of faces when the other face is serving as a reference plane being set to a value in a range of from greater than 0 mm to 0.20 mm.

21. The method of producing a scrub agent according to any one of claims 13 to 20, wherein the molten material comprising a glycerin ester is used in the first step.
